# EUROPEAN PATENT APPLICATION

(11) **EP 2 108 368 A1**
(43) Date of publication of application: **14.10.2009**
(21) Application number: 07816772.3
(22) Date of filing: 07.11.2007
(51) Int. Cl.: A61K 31/337, A61K 31/706, A61K 31/365, A61K 38/16, A61K 47/48, A61P 35/00

(54) **POLY(GLUTAMIC ACID)-DRUG CONJUGATE WITH AN AMINO ACID AS A LINKER**

(30) Priority: 30.12.2006 CN 200610171580
(71) Applicant: Institute of Pharmacology and Toxicology Academy of Military Medical Sciences P.L.A., Beijing 100850 (CN)
(72) Inventor: ZHONG, Bohua, Beijing 100850 (CN); ZHANG, Jianxin, Beijing 100850 (CN); CHEN, Lanfu, Beijing 100850 (CN)
(74) Representative: CAPRI
(86) International application number: PCT/CN2007/003160
(87) International publication number: WO 2008/080291

(57) **Abstract**

A poly(glutamic acid)-drug conjugate with an amino acid as a linker, and nontoxic pharmaceutically acceptable salt thereof, are provided in the present invention. The drug is gemcitabine, paclitaxel or docetaxel. The α-carboxyl group in the amino acid is linked to 5'-hydroxy in gemcitabine molecule or linked to 2'-hydroxy in paclitaxel (docetaxel) molecule via an ester linkage. The α-amino group in the amino acid is linked to the carboxyl group in the poly-(L-glutamic acid) molecule via an amide linkage. The content of gemcitabine in the conjugate is 5-30% in weight, and the content of paclitaxel or docetaxel is 10-40% in weight.

## Description

### Field of the Invention

The present invention relates to a poly(glutamic acid)-drug conjugate with an amino acid as a linker and non-toxic pharmaceutically acceptable salts thereof, wherein the drug is gemcitabine, paclitaxel or docetaxel.

### Background of the Invention

Gemcitabine, paclitaxel and docetaxel are antitumor drugs most commonly clinically used at present, and they have chemical structures as shown below:

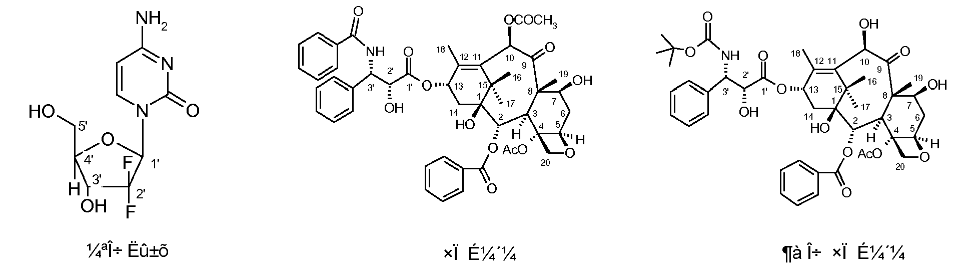

Gemcitabine is a derivative of cytosine nucleoside, and is an antimetabolic type antitumor drug. Results of clinical investigations show that gemcitabine is effective in the treatment of a number of tumors. For pancreatic cancer, gemcitabine can ameliorate clinic symptoms, enhance life quality, and prolong survival time. Hence, gemcitabine is a first line drug for treating pancreatic cancer. The combination of gemcitabine and cisplatin is a preferred approach for treating non-small cell lung carcinoma. In Europe, gemcitabine has also been approved to treat advanced breast cancer. Gemcitabine is also used in the treatment of tumors such as bladder cancer, ovary cancer, lymphoma, and digestive tract cancer. However, gemcitabine has a shorter biological half life and is prone to be metabolized in vivo into an inactive metabolite.

Paclitaxel type drugs are antitumor drugs having tubulin inhibition activities. Paclitaxel and its derivative, docetaxel, can impel tubulin to aggregate into microtubules and bind to the microtubules so as to inhibit the depolymerization of the microtubules, thereby stopping the mitosis of cells. Paclitaxel and docetaxel exhibit significant therapeutic effect for ovarian cancer, breast cancer, non-small cell lung carcinoma, head and neck malignant tumor, and the like, and they can be used in combination with other antitumor drugs such as cisplatin to enhance therapeutic effect. Therefore, paclitaxel and docetaxel have become the most commonly used antitumor drugs. However, paclitaxel and docetaxel have very poor solubility in water.

Although gemcitabine, paclitaxel and docetaxel have been widely clinically used, these drugs as cytotoxic antitumor drugs have side-effects such as marrow inhibition, gastrointestinal response, and the like, and these bad responses are serious.

By coupling an antitumor drug with a macromolecule carrier such as poly(glutamic acid), polyethylene glycol and the like, thereby selectively delivering the drug to a tumor tissue by means of the high permeability of blood vessel tissue of the tumor for the macromolecule conjugate, targeting of the antitumor drug may be enhanced and the toxicity and side effects can be reduced; and the half life of the drug in vivo may be prolonged due to the slow release of the conjugate so that the therapeutic effect can be enhanced.

The coupling bond of the drug-macromolecule conjugate is an important factor affecting the therapeutic effect of the conjugate. Desired coupling bonds should remain stable in blood but can sufficiently release the active drug in the target tissue.

Chinese patent application CN 1217662A and U.S. p atents US 6,262,107, US 6,515,017, US 6,730,699 and US 6,884,817 disclose water-soluble conjugates of paclitaxel or docetaxel with poly(glutamic acid) (poly-(L)-glutamic acid, PLGA). In the above patent publications, paclitaxel or docetaxel directly forms an ester linkage with the carboxyl group of the poly(glutamic acid) molecule under the action of condensation agent, dicyclohexylcarbodiimide.

It can be seen from their chemic al structures that each of gemcitabine, paclitaxel and docetaxel has multiple hydroxyl groups in one molecule. As a consequence, directly coupling the hydroxyl group in the drug molecules with the carboxyl group in the poly(glutamic acid) molecules under the action of a carbodiimide will give a mixture of conjugates with linkages formed from hydroxyl groups at different positions and the carboxyl group. Due to the difference in chemical property or steric hindrance, the coupling bonds formed from different hydroxyl groups of gemcitabine, paclitaxel and docetaxel molecules with the poly(glutamic acid) will possess different chemical stabilities, so that the conjugates will exhibit different bioactivities. According to the techniques of the above-mentioned patents, it is impossible to separate a specific conjugate in which the poly(glutamic acid) is linked to a specific hydroxyl group of the drug molecule.

### Disclosures of the Invention

An object of the present invention is to provide a conjugate formed by coupling gemcitabine, paclitaxel or docetaxel with a poly-(L)-glutamic acid via an amino acid, in which the poly-(L)-glutamic acid is linked to a specific hydroxyl in the drug molecule via an amino acid. By selecting a suitable amino acid linker, controlled release of a free drug can be achieved.

Preferably, in the conjugates formed by coupling said drugs with the poly-(L)-glutamic acid via an amino acid, the α-position carboxyl group in the amino acid molecule forms an ester linkage with the 5'-position hydroxyl group in gemcitabine molecule or 2'-position hydroxyl group in paclitaxel (or docetaxel) molecule, and the α-position amino group in the amino acid molecule forms an amide linkage with the carboxyl group in the poly-(L)-glutamic acid molecules.

Preferably, the amino acid used for coupling the drugs with the poly-(L)-glutamic acids is glycine, (L)-alanine, (L)-leucine, (L)-isoleucine, (L)-valine, (L)-proline, (L)-phenylalanine, (L)-methionine or (L)-glutamine.

Preferably, the poly-(L)-glutamic acid has a molecular weight of from 25,000 to 50,000, and its N-terminal is a free amino or an alkanoylated amino.

Preferably, the content of gemcitabine in the conjugates is in a range of from 5 to 30 wt%, and the content of paclitaxel or docetaxel in the conjugates is in a range of from 10 to 40 wt%.

The present invention further provides non-toxic pharmaceutically acceptable salts, such as inorganic metal ion salts, for example, sodium salt or potassium salt, or organic amine salts of the conjugate formed by coupling gemcitabine, paclitaxel or docetaxel with the poly-(L)-glutamic acid via the amino acid.

The present invention further provides a pharmaceutical composition containing the conjugate formed by coupling gemcitabine, paclitaxel or docetaxel with the poly-(L)-glutamic acid via the amino acid or the non-toxic pharmaceutically acceptable salt thereof as an active component.

The present invention further provides the use of the conjugate of gemcitabine, paclitaxel or docetaxel with the poly-(L)-glutamic acid, the non-toxic pharmaceutically acceptable salts thereof, and the pharmaceutical composition containing the conjugate or the non-toxic pharmaceutically acceptable salts thereof as an active component in the preparation of an antitumor drug.

### The Description of Preferred Embodiments

According to the present invention, the synthesis of the conjugates formed by coupling gemcitabine, paclitaxel or docetaxel with the poly-(L)-glutamic acid via the amino acid may include two steps: the synthesis of an amino acid derivative of the drugs, and the coupling of the amino acid derivative of the drugs with the poly-(L)-glutamic acid.
An amino acid derivative of gemcitabine may be prepared according to the following scheme:

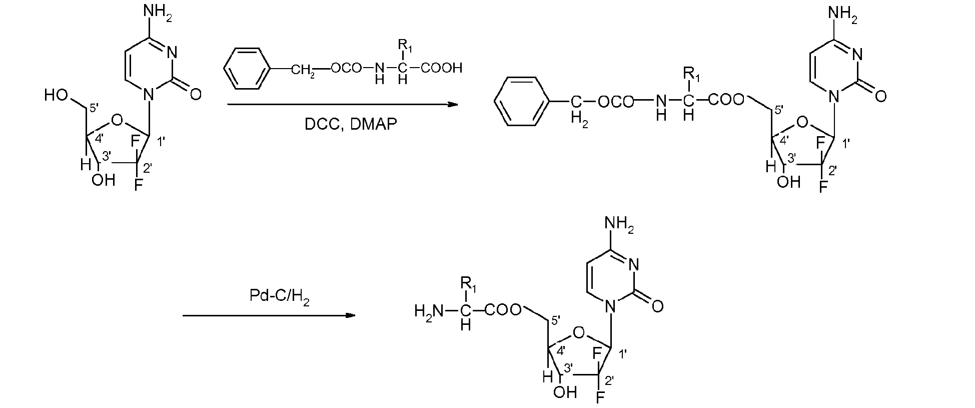

A N-benzyloxycarbonyl amino acid condenses with gemcitabine under the action of dicyclohexylcarbodiimide, and the reaction product is isolated by silica column chromatography to give 5'-O-(N-benzyloxycarbonylcarbamoyl)-gemcitabine, which is catalytically hydrogenat ed to remove the protection group, to give 5'-O-(carbamoyl) -gemcitabine. R₁ is an amino acid side chain such as hydrogen, methyl, isopropyl, isobutyl, 2-methylpropyl, benzyl or the like.

Similarly, a N-benzyloxycarbonyl amino acid condenses with paclitaxel or docetaxel under the action of dicyclohexylcarbodiimide, and the reaction product is isolated by silica column chromatography to give 2'-O-(N-benzyloxycarbonyl -carbamoyl)-paclitaxel or 2'-O-(N-benzyloxycarbonylcarbamoyl)-docetaxel, which is catalytically hyd rogenated to remove the protection group, to give 2'-O-(carbamoyl) -paclitaxel or 2'-O-(carbamoyl)-docetaxel: 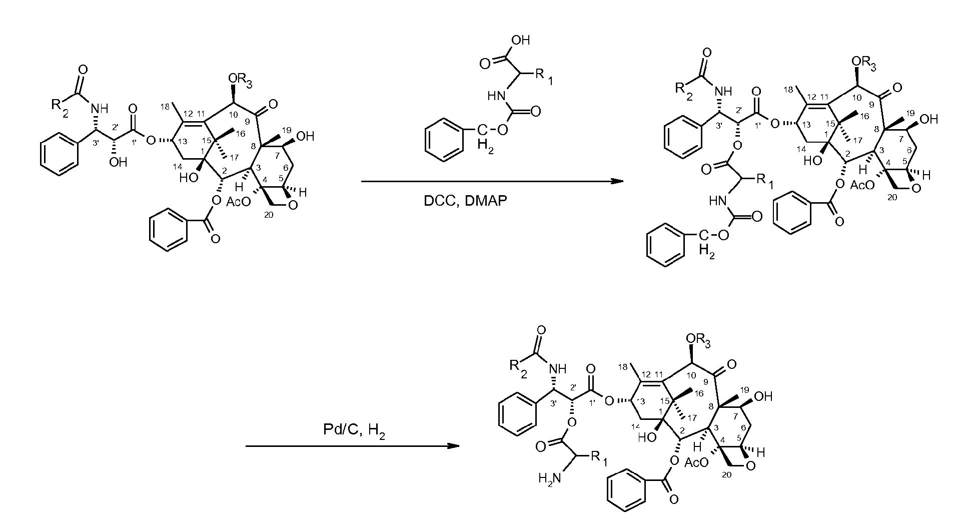 R₁ is an amino acid side chain such as hydrogen, methyl, isopropyl, isobutyl, 2-methylpropyl, benzyl or the like. For paclitaxel, R₂=phenyl, R₃=acetyl; and for docetaxel, R₂=t-butoxy, R₃=H.

The amino group of the amino acid derivatives of gemcitabine, paclitaxel or docetaxel may directly condense with the carboxyl group of the poly-(L)-glutamic acid under the action of dicyclohexylcarbodiimide, to form an amide linkage:

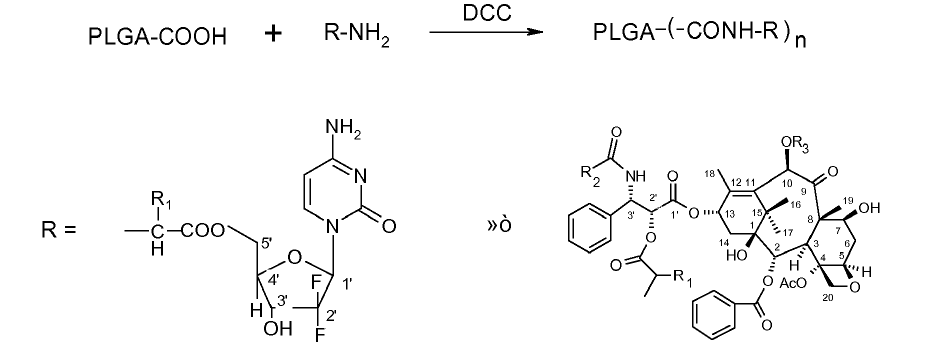

The amino acid derivatives of gemcitabine, paclitaxel or docetaxel may also be coupled with the N-acetyl-poly-(L)-glutamic acid through an active ester route:

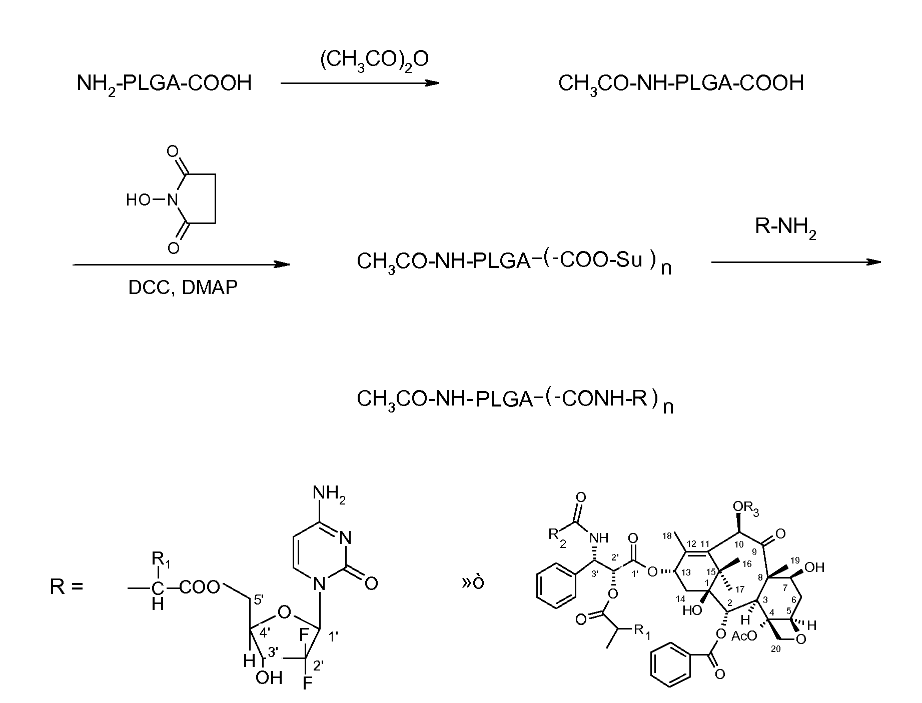

The poly-(L)-glutamic acid reacts first with acetic anhydride, to form N-acetylated poly-(L)-glutamic acid. Then the carboxyl group of the N-acetylated poly-(L)-glutamic acid forms an active ester with N-hydroxysuccimide under the action of dicyclohexylcarbodiimide. Finally, the active ester reacts with the amino group of the amino acid derivatives of gemcitabine, paclitaxel or docetaxel to form an amide linkage.

The following examples will further illustrate the present invention, but cannot be interpreted as limitation to the scope of the invention.

Example 1 Preparation of [poly-(L)-glutamic acid]-[5'-O-(glycyl)-gemcitabine] conjugate (I₁)

### 1.1 Synthesis of 5'-O-(glycyl)-gemcitabine

0.4 g (2 mmol) of N-benzyloxycarbonyl-glycine and 0.02 g of 4-dimethylamino pyridine were dissolved in 2 ml of dry dimethylformamide and cooled to 5 °C, and then 0.6 g (2 mmol) of gemcitabine was added thereto. While stirring, 0.42 g (2 mmol) of dicyclohexylcarbodiimide was added thereto, and the mixture was allowed to react at 5 °C with stirring for 5 hours. White precipitates were filtered off, and the filtrate was concentrated under vacuum. Then the residue was isolated by silica column chromatography with ethyl acetate/petroleum ether/methanol (5:5:0.1) as an eluant. The desired fractions were collected and then evaporated to dryness under vacuum, to give 0.34 g of 5'-O-(N-benzyloxycarbonylglycyl)-gemcitabine.

0.34 g of 5'-O-(N-benzyloxycarbonylglycyl)-gemcitabine was dissolved in 5 ml of dioxane, 0.1 g of 5% palladium on carbon was added thereto, and the mixture was stirred under 1 atm of hydrogen gas for 4 hours. After filtering off the catalyst, the filtrate was concentrated under vacuum, and then the residue was isolated by silica column chromatography with ethyl acetate/petroleum ether/methanol (8:2:0.5) as an eluant. The desired fractions were collected and then evaporated to dryness under vacuum, to give 0.23 g of 5'-O-(glycyl)-gemcitabine. ¹HNMR: δ (ppm, DMF-d₇): 8.02(d, 1H); 6.32(d, 1H); 6.15(d, 1H); 4.48 (m, 1H); 4.19(m,1H); 3.96(m, 2H); 3.88(m, 2H).

### 1.2 Synthesis of [poly-(L)-glutamic acid]-[5'-O-(glycyl)-gemcitabine] conjugate (I₁)

2 g of poly-(L)-glutamate sodium (having a molecular weight ranging from 25,000 to 50,000, available from Sigma Corp. ) was dissolved in water. The solution was adjusted with hydrochloric acid to acidic pH, dialyzed with double distilled water, and then lyophilized to give 1.5 g of poly-(L)-glutamic acid.

100 mg of poly-(L)-glutamic acid was dissolved in 2 ml of dry dimethylformamide, and 32 mg (0.1 mmol) of 5'-O-(glycyl)-gemcitabine and 20.6 mg (0.1 mmol) of dicyclohexylcarbodiimide were added thereto. The mixture was allowed to react at room temperature with stirring for 6 hours. After filtering, the filtrate was evaporated under vacuum to dryness, and the residue was dissolved in sodium bicarbonate solution, dialyzed with double distilled water, and lyophilized to give 119 mg of I₁. 20 mg of I₁ was hydrolyzed with a sodium hydroxide solution, and then the content of free gemcitabine was found by a HPLC method as 3.8 mg. Thus, the content of gemcitabine in the conjugate I₁ was calculated as 19wt%.

### Example 2 Preparation of [N-acetyl-poly-(L)-glutamic acid]-[5'-O-(glycyl) -gemcitabine] conjugate (I₂)

2 g of poly-(L)-glutamate sodium was dissolved in water, 0.5 ml of acetic anhydride was added thereto, and the mixture was stirred at room temperature for 2 hours. Then the reaction mixture was adjusted with hydrochloric acid to acidic pH, dialyzed with double distilled water, and lyophilized to give 1.4 g of N-acetyl-poly-(L)-glutamic acid.

100 mg of N-acetyl-poly-(L)-glutamic acid was dissolved in 2 ml of dry dimethylformamide, and 14 mg (0.1 mmol) of N-hydroxysuccimide and 20.6 mg (0.1 mmol) of dicyclohexylcarbodiimide were added thereto. The mixture was allowed to react at room temperature with stirring for 6 hours. After filtering, 32 mg (0.1 mmol) of 5'-O-(glycyl)-gemcitabine was added to the filtrate, and the mixture was stirred over night. Then the reaction mixture was evaporated to dryness under vacuum, and the residue was dissolved in sodium bicarbonate solution, dialyzed with double distilled water, and lyophilized to give106 mg of I₂.

20 mg of I₂ was hydrolyzed with a sodium hydroxide solution, and then the content of free gemcitabine was found by a HPLC method as 3.5mg. Thus, the content of gemcitabine in the conjugate I₂ was calculated as 17.5wt%.

### Example 3 Preparation of [poly-(L)-glutamic acid]-[2'-O-(glycyl)-paclitaxel] conjugate (I₃)

### 3.1 Synthesis of 2'-O-(glycyl)-paclitaxel

0.2 g (1 mmol) of N -benzyloxycarbonyl-glycine and 0.01 g of 4-dimethylamino pyridine were dissolved in 2 ml of dry dimethylformamide and cooled to 5 °C, and then 0.85 g (1 mmol) of paclitaxel was added thereto. While stirring, 0.21 g (1 mmol) of dicyclohexylcarbodiimide was added thereto, and the mixture was allowed to react at 5 °C with stirring for 5 hours. White precipitates were filtered off, and the filtrate was concentrated under vacuum. Then the residue was isolated by silica column chromatography with ethyl acetate/petroleum ether/methanol (5:5:0.1) as an eluant. The desired fractions were collected, and then evaporated to dryness under vacuum, to give 0.79 g of 2'-O-(N-benzyloxycarbonylglycyl)-paclitaxel.
0.79 g of 2'-O-(N-benzyloxycarbonylglycyl)-paclitaxel was dissolved in 5 ml of dioxane, 0.1g of 5% palladium on carbon was added thereto, and the mixture was stirred under 1 atm of hydrogen gas for 4 hours. After filtering off the catalyst, the filtrate was concentrated under vacuum, and then the residue was isolated by silica column chromatography with ethyl acetate/petroleum ether/methanol (8:2:0.5) as an eluant. The desired fractions were collected, and then evaporated to dryness under vacuum, to give 0.62 g of 2'-O-(glycyl)-paclitaxel. ¹HNMR: δ (ppm, DMSO-d₆): 9.26(d, 1H); 8.41(brs, 2H); 8.02-7.20(m, 15H); 6.32(s, 1H); 5.83(t, 1H); 5.59(t, 1H); 5.45 (d, 1H); 5.41(d, 1H); 4.92(dd, 1H); 4.90(brs, 1H); 4.63(s, 1H); 4.11(dd, 1H); 3.95-4.06 (m, 3H); 3.57(d, 1H); 2.34(m, 1H); 2.24(s, 3H); 2.10(s, 3H); 1.86(s, 3H); 1.80(dd, 1H); 1.64(t, 1H); 1.52(s, 3H); 1.06(s, 3H); 1.02(s, 3H).

### 3.2 Synthesis of [poly-(L)-glutamic acid]-[2'-O-(glycyl)-paclitaxel] conjugate (I₃)

100 mg of poly-(L)-glutamic acid was dissolved in 2 ml of dry dimethylformamide, and 46mg (0.05 mmol) of 2'-O-(glycyl)-paclitaxel and 11mg (0.05 mmol) of dicyclohexylcarbodiimide were added thereto. The mixture was allowed to react at room temperature with stirring for 6 hours. After filtering, the filtrate was evaporated under vacuum to dryness, and the residue was dissolved in sodium bicarbonate solution and then extracted with methylene chloride for three times. The aqueous layer was dialyzed with double distilled water, and lyophilized to give 126mg of I₃. 20mg of I₃ was hydrolyzed with a sodium hydroxide solution, then the reaction mixture was extracted with methylene chloride for three times. The combined extracts were washed in turn with 1M hydrochloric acid, 1M sodium bicarbonate solution and saturated NaCl solution, and the organic layer was dried and then evaporated under vacuum to dryness, to give 5.9mg of free paclitaxel. Thus the content of paclitaxel in the conjugate I₃ was calculated as 29.5wt%.

### Example 4 Preparation of [N-acetyl-poly-(L)-glutamic acid]-[2'-O-(glycyl) -paclitaxel] conjugate (I₄)

100 mg of N-acetyl-poly-(L)-glutamic acid was dissolved in 2 ml of dry dimethylformamide, and 7 mg (0.05 mmol) of N-hydroxysuccimide and 11 mg (0.05 mmol) of dicyclohexylcarbodiimide was added thereto. The mixture was allowed to react at room temperature with stirring for 6 hours. After filtering, 46mg (0.05 mmol) of 2'-O-(glycyl)-paclitaxel was added to the filtrate, and the mixture was stirred over night. Then the reaction mixture was evaporated under vacuum to dryness, and the residue was dissolved in sodium bicarbonate solution and extracted with methylene chloride for three times. The aqueous layer was dialyzed with double distilled water and then lyophilized to give117mg of I₄. 20mg of I₄ was hydrolyzed with a sodium hydroxide solution, then the reaction mixture was extracted with methylene chloride for three times. The combined extracts were washed in turn with 1M hydrochloric acid, 1M sodium bicarbonate solution and saturated NaCl solution, and the organic layer was dried and then evaporated under vacuum to dryness, to give 5.4mg of free paclitaxel. Thus, the content of paclitaxel in the conjugate I₄ was calculated as 27wt%.

### Example 5 Preparation of [poly-(L)-glutamic acid]-[2'-O-(glycyl)-docetaxel] conjugate (I₅)

### 5.1 Synthesis of 2'-O-(glycyl)-docetaxel

0.2g (1 mmol) of N-benzyloxycarbonyl-glycine and 0.01g of 4-dimethylamino pyridine were dissolved in 2 ml of dry dimethylformamide and cooled to 5°C. Then 0.81 g (1 mmol) of docetaxel was added thereto. While stirring, 0.21g (1 mmol) of dicyclohexylcarbodiimide was added thereto, and the mixture was allowed to react at 5°C with stirring for 5 hours. White precipitates were filtered off, and the filtrate was concentrated under vacuum. Then the residue was isolated by silica column chromatography with ethyl acetate/petroleum ether/methanol (5:5:0.1) as an eluant. The desired fractions were collected, and then evaporated to dryness under vacuum, to give 0.68 g of 2'-O-(N-benzyloxycarbonylglycyl)-docetaxel.

0.68 g of 2'-O-(N-benzyloxycarbonylglycyl)-docetaxel was dissolved in 5 ml of dioxane, 0.1g of 5% palladium on carbon was added thereto, and the mixture was stirred under 1 atm of hydrogen gas for 4 hours. After filtering off the catalyst, the filtrate was concentrated under vacuum, and then the residue was isolated by silica column chromatography with ethyl acetate/petroleum ether/methanol (8:2:0.5) as an eluant. The desired fractions were collected, and then evaporated to dryness under vacuum, to give 0.42 g of 2'-O-(glycyl)-docetaxel. ¹HNMR: δ (ppm, DMSO-d₆): 9.25(d, 1H); 8.48(brs, 2H); 8.05-7.32(m, 10H); 6.38(s, 1H); 5.80(t, 1H); 5.57(t, 1H); 5.42(d, 1H); 5.36(d, 1H); 4.94(dd, 1H); 4.82(brs, 1H); 4.61(s, 1H); 4.14(dd, 1H); 3.95-4.06 (m, 3H); 3.57(d, 1H); 2.34(m, 1H); 2.24(s, 3H); 1.86(s, 3H); 1.80(dd, 1H); 1.64(t, 1H); 1.52(s, 3H); 1.40(s, 9H); 1.06(s, 3H); 1.02(s, 3H).

### 5.2 Synthesis of [poly-(L)-glutamic acid]-[2'-O-(glycyl)-docetaxel] conjugate (I₅)

100 mg of poly-(L)-glutamic acid was dissolved in 2 ml of dry dimethylformamide, and 43 mg (0.05 mmol) of 2'-O-(glycyl)-paclitaxel and 11 mg (0.05 mmol) of dicyclohexylcarbodiimide were added thereto. The mixture was allowed to react at room temperature with stirring for 6 hours. The mixture was filtered, and the filtrate was evaporated under vacuum to dryness. The residue was dissolved in sodium bicarbonate solution and extracted with methylene chloride for three times. The aqueous layer was dialyzed with double distilled water, and lyophilized to give 126mg of I₅. 20mg of I₅ was hydrolyzed with a sodium hydroxide solution, then the reaction mixture was extracted with methylene chloride for three times. The combined extracts were washed in turn with 1M hydrochloric acid, 1M sodium bicarbonate solution and saturated NaCl solution, and the organic layer was dried and then evaporated under vacuum to dryness, to give 5.6mg of free docetaxel. Thus, the content of docetaxel in the conjugate I₅ was calculated as 28wt%.

### Example 6 Preparation of [N-acetyl-poly-(L)-glutamic acid]-[2'-O-(glycyl) -docetaxel] conjugate (I₆)

I₆ was prepared by following the procedure as described in Example 4 except for replacing 2'-O-(glycyl)-paclitaxel with 2'-O-(glycyl)-docetaxel in the reaction with the active ester of N-acetyl-poly-(L)-glutamic acid. The content of docetaxel in the conjugate I₆ was found as 21wt%.

### Example 7 Preparation of [poly-(L)-glutamic acid]-[5'-O-(L-alanyl)-gemcitabine] conjugate (I₇)

5'-O-(N-benzyloxycarbonyl-L-alanyl)-gemcitabine was prepared by following the procedure as described in Example 1.1 except for replacing N-benzyloxycarbonyl-glycine with N-benzyloxycarbonyl-(L)-alanine in the reaction with gemcitabine. After catalytically hydrogenating, 5'-O-(L-alanyl)-gemcitabine was obtained. ¹HNMR: δ (ppm, DMF-d₇): 7.94(d, 1H); 6.28(d, 1H); 6.13(d, 1H); 4.45 (m, 1H); 4.16(m, 1H); 3.88(m, 2H); 3.54(q, 1H); 1.26(d, 3H).

I₇ was prepared by following the procedure as described in Example 1.2 except for replacing 5'-O-(glycyl)-gemcitabine with 5'-O-(L-alanyl)-gemcitabine in the condensation with the poly-(L)-glutamic acid under the action of dicyclohexylcarbodiimide. The content of gemcitabine in the conjugate I₇ was found as 16.5 wt%.

### Example 8 Preparation of [N-acetyl-poly-(L)-glutamic acid]-[5'-O-(L-alanyl) -gemcitabine] conjugate (I₈)

I₈ was prepared by following the procedure as described in Example 2 except for replacing 5'-O-(glycyl)-gemcitabine with 5'-O-(L-alanyl)-gemcitabine in the reaction with the active ester of N-acetyl-poly-(L)-glutamic acid. The content of gemcitabine in the conjugate I₈ was found as 15wt%.

### Example 9 Preparation of [poly-(L)-glutamic acid]-[2'-O-(L-alanyl)-paclitaxel] conjugate (I₉)

### 9.1 Synthesis of 2'-O-(L-alanyl)-paclitaxel

2'-O-(N-benzyloxycarbonyl-L-alanyl)-paclitaxel was prepared by following the procedure as described in Example 3.1 except for replacing N-benzyloxycarbonyl-glycine with N-benzyloxycarbonyl-(L)-alanine in the condensation reaction with paclitaxel. Catalytic hydrogenation was performed to remove the protection group, to give 2'-O-(L-alanyl)-paclitaxel. ¹HNMR: δ (ppm, DMSO-d₆): 9.20(d, 1H); 8.02-7.22(m, 15H); 6.31(s, 1H); 5.86(t, 1H); 5.61(t, 1H); 5.45 (d, 1H); 5.38(d, 1H); 4.92(dd, 1H); 4.90(brs, 1H); 4.64(s, 1H); 4.10(dd, 1H); 4.05(d, 1H); 4.02(d, 1H); 3.59(d, 1H); 3.54(q, 1H); 2.35(m, 1H); 2.26(s, 3H); 2.12(s, 3H); 1.86(s, 3H); 1.64(t, 1H); 1.52(s, 3H); 1.24(d, 3H); 1.06(s, 3H); 1.02(s, 3H).

### 9.2 Synthesis of [poly-(L)-glutamic acid]-[2'-O-(alanyl)-paclitaxel] conjugate (I₉)

I₆ was prepared by following the procedure as described in Example 3.2 except for replacing 2'-O-(glycyl)-paclitaxel with 2'-O-(L-alanyl)-paclitaxel in the condensation reaction with the poly-(L)-glutamic acid under the action of dicyclohexylcarbodiimide. The content of paclitaxel in the conjugate I₆ was found as 25.5 wt%.

### Example 10 Preparation of [N-acetyl-poly-(L)-glutamic acid]-[2'-O-(L-alanyl) -paclitaxel] conjugate (I₁₀)

I₁₀ was prepared by following the procedure as described in Example 4 except for replacing 2'-O-(glycyl)-paclitaxel with 2'-O-(L-alanyl)-paclitaxel in the reaction with the active ester of N-acetyl-poly-(L)-glutamic acid. The content of paclitaxel in the conjugate was found as 23.6 wt%.

### Example 11 Preparation of [poly-(L)-glutamic acid]-[2'-O-(L-alanyl) -docetaxel] conjugate (I₁₁)

### 11.1 Synthesis of 2'-O-(L-alanyl)-docetaxel

2'-O-(N-benzyloxycarbonyl-L-alanyl)-docetaxel was prepared by following the procedure as described in Example 5.1 except for replacing N-benzyloxycarbonyl-glycine with N-benzyloxycarbonyl-(L)-alanine in the condensation reaction with docetaxel. Catalytic hydrogenation was performed to remove the protection group, to give 2'-O-(L-alanyl) -docetaxel. ¹HNMR:δ (ppm, DMSO-d₆): 9.25(d, 1H); 8.48(brs, 2H); 8.05-7.32(m, 10H); 6.38(s, 1H); 5.80(t, 1H); 5.57(t, 1H); 5.42(d, 1H); 5.36(d, 1H); 4.94(dd, 1H); 4.82(brs, 1H); 4.61(s, 1H); 4.14(dd, 1H); 4.01(d, 1H); 4.04(d, 1H); 3.57(d, 1H); 3.55(q, 1H); 2.34(m, 1H); 2.24(s, 3H); 1.86(s, 3H); 1.80(dd, 1H); 1.64(t, 1H); 1.52(s, 3H); 1.40(s, 9H); 1.22(d, 3H); 1.06(s, 3H); 1.02(s, 3H).

### 11.2 Synthesis of [poly-(L)-glutamic acid]-[2'-O-(alanyl)-docetaxel] conjugate (I₁₁)

I₁₁ was prepared by following the procedure as described in Example 3.2 except for replacing 2'-O-(glycyl)-paclitaxel with 2'-O-(L-alanyl)-docetaxel in the condensation reaction with the poly-(L)-glutamic acid under the action of dicyclohexylcarbodiimide. The content of docetaxel in the conjugate I₁₁ was found as 23.4 wt%.

### Example 12 Preparation of [N-acetyl-poly-(L)-glutamic acid]-[2'-O-(L-alanyl) -docetaxel] conjugate (I₁₂)

I₁₂ was prepared by following the procedure as described in Example 4 except for replacing 2'-O-(glycyl)-paclitaxel with 2'-O-(L-alanyl)-docetaxel in the reaction with the active ester of N-acetyl-poly-(L)-glutamic acid. The content of paclitaxel in the conjugate was found as 23.1 wt%.

### Example 13 Preparation of [poly-(L)-glutamic acid]-[5'-O-(L-isoleucyl) -gemcitabine] conjugate (I₁₃)

5'-O-(N-benzyloxycarbonyl-L-isoleucyl)-gemcitabine was prepared by following the procedure as described in Example 1.1 except for replacing N-benzyloxycarbonyl-glycine with N-benzyloxycarbonyl-(L)-isoleucine in the reaction with gemcitabine. After catalytically hydrogenating, 5'-O-(L-isoleucyl)-gemcitabine was obtained. ¹HNMR: δ (ppm, DMF-d₇): 7.94(d, 1H); 6.28(d, 1H); 6.13(d, 1H); 4.45 (m, 1H); 4.16(m, 1H); 3.88(m, 2H); 3.54(q, 1H); 1.35(m, 1H); 1.03(m, 2H); 0.82(d, 3H); 0.64(t, 3H).

I₁₃ was prepared by following the procedure as described in Example 1.2 except for replacing 5'-O-(glycyl)-gemcitabine with 5'-O-(L-isoleucyl)-gemcitabine in the condensation reaction with the poly-(L)-glutamic acid under the action of dicyclohexylcarbodiimide. The content of gemcitabine in the conjugate I₁₃ was found as 14.2 wt%.

### Example 14 Preparation of [N-acetyl-poly-(L)-glutamic acid]-[5'-O-(L-isoleucyl) -gemcitabine] conjugate (I₁₄)

I₈ was prepared by following the procedure as described in Example 2 except for replacing 5'-O-(glycyl)-gemcitabine with 5'-O-(L-isoleucyl)-gemcitabine in the reaction with the active ester of N-acetyl-poly-(L)-glutamic acid. The content of gemcitabine in the conjugate I₁₄ was found as 13.4 wt%.

### Example 15 Preparation of [poly-(L)-glutamic acid]-[2'-O-(L-isoleucyl) -paclitaxel] conjugate (I₁₅)

### 15.1 Synthesis of 2'-O-(L-isoleucyl)-paclitaxel

2'-O-(N-benzyloxycarbonyl-L-isoleucyl)-paclitaxel was prepared by following the procedure as described in Example 3.1 except for replacing N-benzyloxycarbonyl-glycine with N-benzyloxycarbonyl-(L)-isoleucine in the condensation reaction with paclitaxel. Catalytic hydrogenation was performed to remove the protection group, to give 2'-O-(L-isoleucyl)-paclitaxel. ¹HNMR: δ (ppm, DMSO-d₆): 9.23(d, 1H); 8.02-7.22(m, 15H); 6.31(s, 1H); 5.86(t, 1H); 5.61(t, 1H); 5.45 (d, 1H); 5.37(d, 1H); 4.92(dd, 1H); 4.90(brs, 1H); 4.64(s, 1H); 4.12(dd, 1H); 4.05(d, 1H); 4.02(d, 1H); 3.64(m, 1H); 3.59(d, 1H); 2.35(m, 1H); 2.26(s, 3H); 2.12(s, 3H); 1.86(s, 3H); 1.64(t, 1H); 1.52(s, 3H); 1.35(m, 1H); 1.06(s, 3H); 1.03(m, 2H); 1.02(s, 3H); 0.82(d, 3H); 0.64(t, 3H).

15.2 Synthesis of [poly-(L)-glutamic acid]-[2'-O-(isoleucyl)-paclitaxel] conjugate (I₁₅)

I₁₅ was prepared by following the procedure as described in Example 3.2 except for replacing 2'-O-(glycyl)-paclitaxel with 2'-O-(L-isoleucyl)-paclitaxel in the condensation with the poly-(L)-glutamic acid under the action of dicyclohexylcarbodiimide. The content of paclitaxel in the conjugate I₁₅ was found as 21.3 wt%.

### Example 16 Preparation of [N-acetyl-poly-(L)-glutamic acid]-[2'-O-(L-isoleucyl) -paclitaxel] conjugate (I₁₆)

I₁₆ was prepared by following the procedure as described in Example 4 except for replacing 2'-O-(glycyl)-paclitaxel with 2'-O-(L-isoleucyl)-paclitaxel in the reaction with the active ester of N-acetyl-poly-(L)-glutamic acid. The content of paclitaxel in the conjugate was found as 20.8 wt%.

### Example 17 Preparation of [poly-(L)-glutamic acid]-[2'-O-(L-isoleucyl) -docetaxel] conjugate (I₁₇)

### 17.1 Synthesis of 2'-O-(L-isoleucyl)-docetaxel

2'-O-(N-benzyloxycarbonyl-L-isoleucyl)-docetaxel was prepared by following the procedure as described in Example 5.1 except for replacing N-benzyloxycarbonyl-glycine with N-benzyloxycarbonyl-(L)-isoleucine in the condensation with docetaxel. Catalytic hydrogenation was performed to remove the protection group, to give 2'-O-(L-isoleucyl) -docetaxel. ¹HNMR: δ (ppm, DMSO-d₆): 9.25(d, 1H); 8.48(brs, 2H); 8.05-7.32(m, 10H); 6.38(s, 1H); 5.80(t, 1H); 5.57(t, 1H); 5.42(d, 1H); 5.36(d, 1H); 4.94(dd, 1H); 4.82(brs, 1H); 4.61(s, 1H); 4.14(dd, 1H); 4.01(d, 1H); 4.04(d, 1H); 3.64(m, 1H); 3.57(d, 1H); 2.34(m, 1H); 2.24(s, 3H); 1.86(s, 3H); 1.80(dd, 1H); 1.64(t, 1H); 1.52(s, 3H); 1.40(s, 9H); 1.35(m, 1H); 1.06(s, 3H); 1.03(m, 2H); 1.02(s, 3H); 0.82(d, 3H); 0.64(t, 3H).

17.2 Synthesis of [poly-(L)-glutamic acid]-[2'-O-(isoleucyl)-docetaxel] conjugate (I₁₇)

I₁₇ was prepared by following the procedure as described in Example 3.2 except for replacing 2'-O-(glycyl)-paclitaxel with 2'-O-(L-isoleucyl)-docetaxel in the condensation with the poly-(L)-glutamic acid under the action of dicyclohexylcarbodiimide. The content of docetaxel in the conjugate I₁₇ was found as 22.1wt%.

### Example 18 Preparation of [N-acetyl-poly-(L)-glutamic acid]-[2'-O-(L-isoleucyl) -docetaxel] conjugate (I₁₈)

I₁₈ was prepared by following the procedure as described in Example 4 except for replacing 2'-O-(glycyl)-paclitaxel with 2'-O-(L-isoleucyl)-docetaxel in the reaction with the active ester of the N-acetyl-poly-(L)-glutamic acid. The content of paclitaxel in the conjugate was found as 21.5wt%.

### Example 19 Preparation of [poly-(L)-glutamic acid]-[5'-O-(L-valyl)-gemcitabine] conjugate (I₁₉)

5'-O-(N-benzyloxycarbonyl-L-valyl)-gemcitabine was prepared by following the procedure as described in Example 1.1 except for replacing N-benzyloxycarbonyl-glycine with N-benzyloxycarbonyl-(L)-valine in the reaction with gemcitabine. After catalytically hydrogenating, 5'-O-(L-valyl)-gemcitabine was obtained. ¹HNMR: δ (ppm, DMF-d₇): 7.94(d, 1H); 6.28(d, 1H); 6.13(d, 1H); 4.45 (m, 1H); 4.16(m, 1H); 3.69(m, 1H); 3.88(m, 2H); 2.25(m, 1H); 0.91(m, 6H).

I₁₉ was prepared by following the procedure as described in Example 1.2 except for replacing 5'-O-(glycyl)-gemcitabine with 5'-O-(L-valyl)-gemcitabine in the condensation with the poly-(L)-glutamic acid under the action of dicyclohexylcarbodiimide. The content of gemcitabine in the conjugate I₁₉ was found as 16.2 wt%.

### Example 20 Preparation of [N-acetyl-poly-(L)-glutamic acid]-[5'-O-(L-valyl) -gemcitabine] conjugate (I₂₀)

I₂₀ was prepared by following the procedure as described in Example 2 except for replacing 5'-O-(glycyl)-gemcitabine with 5'-O-(L-valyl)-gemcitabine in the reaction with the active ester of the N-acetyl-poly-(L)-glutamic acid. The content of gemcitabine in the conjugate I₂₀ was found as 15wt%.

### Example 21 Preparation of [poly-(L)-glutamic acid]-[2'-O-(L-valyl) -paclitaxel] conjugate (I₂₁)

### 21.1 Synthesis of 1'-O-(L-valyl)-paclitaxel

2'-O-(N-benzyloxycarbonyl-L-valyl)-paclitaxel was prepared by following the procedure as described in Example 3.1 except for replacing N-benzyloxycarbonyl-glycine with N-benzyloxycarbonyl-(L)-valine in the condensation with paclitaxel. A catalytic hydrogenation was performed to remove the protection group, to give 2'-O-(L-valyl) -paclitaxel. ¹HNMR: δ (ppm, DMSO-d₆): 9.23(d, 1H); 8.02-7.22(m, 15H); 6.31(s, 1H); 5.86(t, 1H); 5.61(t, 1H); 5.45 (d, 1H); 5.40(d, 1H); 4.92(dd, 1H); 4.90(brs, 1H); 4.64(s, 1H); 4.10(dd, 1H); 4.05(d, 1H); 4.02(d, 1H); 3.69(m, 1H); 3.59(d, 1H); 2.35(m, 1H); 2.26(s, 3H); 2.21(m, 1H); 2.12(s, 3H); 1.86(s, 3H); 1.64(t, 1H); 1.52(s, 3H); 1.06(s, 3H); 1.02(s, 3H); 0.91(m, 6H).

### 21.2 Synthesis of [poly-(L)-glutamic acid]-[2'-O-(valyl)-paclitaxel] conjugate (I₂₁)

I₂₁ was prepared by following the procedure as described in Example 3.2 except for replacing 2'-O-(glycyl)-paclitaxel with 2'-O-(L-valyl)-paclitaxel in the condensation with the poly-(L)-glutamic acid under the action of dicyclohexylcarbodiimide. The content of paclitaxel in the conjugate I₂₁ was found as 25.5 wt%.

### Example 22 Preparation of [N-acetyl-poly-(L)-glutamic acid]-[2'-O-(L-valyl) -paclitaxel] conjugate (I₂₂)

I₂₂ was prepared by following the procedure as described in Example 4 except for replacing 2'-O-(glycyl)-paclitaxel with 2'-O-(L-valyl)-paclitaxel in the reaction with the active ester of N-acetyl-poly-(L)-glutamic acid. The content of paclitaxel in the conjugate was found as 23.6 wt%.

### Example 23 Preparation of [poly-(L)-glutamic acid]-[2'-O-(L-valyl)-docetaxel] conjugate (I₂₃)

### 23.1 Synthesis of 2'-O-(L-valyl)-docetaxel

2'-O-(N-benzyloxycarbonyl-L-valyl)-docetaxel was prepared by following the procedure as described in Example 5.1 except for replacing N-benzyloxycarbonyl -glycine with N-benzyloxycarbonyl-(L)-valine in the condensation reaction with docetaxel. Catalytic hydrogenation was performed to remove the protection group, to give 2'-O-(L-valyl)-docetaxel. ¹HNMR: δ (ppm, DMSO-d₆): 9.25(d, 1H); 8.48(brs, 2H); 8.05-7.32(m, 10H); 6.38(s, 1H); 5.80(t, 1H); 5.57(t, 1H); 5.42(d, 1H); 5.36(d, 1H); 4.94(dd, 1H); 4.82(brs, 1H); 4.61(s, 1H); 4.14(dd, 1H); 4.01(d, 1H); 4.04(d, 1H); 3.69(m, 1H); 3.57(d, 1H); 2.34(m, 1H); 2.25(m, 1H); 2.24(s, 3H); 1.86(s, 3H); 1.80(dd, 1H); 1.64(t, 1H); 1.52(s, 3H); 1.40(s, 9H); 1.06(s, 3H); 1.02(s, 3H); 0.91(m, 6H).

### 23.2 Synthesis of [poly-(L)-glutamic acid]-[2'-O-(valyl)-docetaxel] conjugate (I₂₃)

I₂₃ was prepared by following the procedure as described in Example 3.2 except for replacing 2'-O-(glycyl)-paclitaxel with 2'-O-(L-valyl)-docetaxel in the condensation with the poly-(L)-glutamic acid under the action of dicyclohexylcarbodiimide. The content of docetaxel in the conjugate I₂₃ was found as 23.4 wt%.

### Example 24 Preparation of [N-acetyl-poly-(L)-glutamic acid]-[2'-O-(L-valyl) -docetaxel] conjugate (I₂₄)

I₂₄ was prepared by following the procedure as described in Example 4, except for replacing 2'-O-(glycyl)-paclitaxel with 2'-O-(L-valyl)-docetaxel in the reaction with the active ester of the N-acetyl-poly-(L)-glutamic acid. The content of paclitaxel in the conjugate was found as 23.1wt%.

### Example 25 Preparation of [poly-(L)-glutamic acid]-[5'-O-(L-phenylalanyl) -gemcitabine] conjugate (I₂₅)

5'-O-(N-benzyloxycarbonyl-L-phenylalanyl)-gemcitabine was prepared by following the procedure as described in Example 1.1, except for replacing N-benzyloxycarbonyl-glycine with N-benzyloxycarbonyl-(L)-phenylalanine in the reaction with gemcitabine. After catalytically hydrogenati ng, 5'-O-(L-phenylalanyl) -gemcitabine was obtained. ¹HNMR: δ (ppm, DMF-d₇): 7.94(d, 1H); 7.23(m, 5H); 6.28(d, 1H); 6.13(d, 1H); 4.52(m, 1H); 4.45 (m, 1H); 4.16(m, 1H); 3.88(m, 2H); 3.15(dd, 1H); 3.01(dd, 1H).

I₂₅ was prepared by following the procedure as described in Example 1.2, except for replacing 5'-O-(glycyl)-gemcitabine with 5'-O-(L-phenylalanyl)-gemcitabine in the condensation with the poly-(L)-glutamic acid under the action of dicyclohexylcarbodiimide. The content of gemcitabine in the conjugate I₂₅ was found as 17.2wt%.

### Example 26 Preparation of [N-acetyl-poly-(L)-glutamic acid]-[5'-O -(L-phenylalanyl)-gemcitabine] conjugate (I₂₆)

I₂₆ was prepared by following the procedure as described in Example 2, except for replacing 5'-O-(glycyl)-gemcitabine with 5'-O-(L-phenylalanyl)-gemcitabine in the reaction with the active ester of the N-acetyl-poly-(L)-glutamic acid. The content of gemcitabine in the conjugate I₂₆ was found as 16.5wt%.

### Example 27 Preparation of [poly-(L)-glutamic acid]-[2'-O-(L-phenylalanyl) -paclitaxel] conjugate (I₂₇)

### 27.1 Synthesis of 2'-O-(L-phenylalanyl)-paclitaxel

2'-O-(N-benzyloxycarbonyl-L-phenylalanyl)-paclitaxel was prepared by following the procedure as described in Example 3.1, except for replacing N-benzyloxy -carbonyl-glycine with N-benzyloxycarbonyl-(L)-phenylalanine in the condensation with paclitaxel. Catalytic hydrogenation was performed to remove the protection group, to give 2'-O-(L-phenylalanyl)-paclitaxel. ¹HNMR: δ (ppm, DMSO-d₆): 9.23(d, 1H); 8.02-7.22(m, 20H); 6.31(s, 1H); 5.86(t, 1H); 5.61(t, 1H); 5.45 (d, 1H); 5.40(d, 1H); 4.92(dd, 1H); 4.90(brs, 1H); 4.64(s, 1H); 4.52(m, 1H); 4.10(dd, 1H); 4.05(d, 1H); 4.02(d, 1H); 3.59(d, 1H); 3.15(dd, 1H); 3.01(dd, 1H); 2.35(m, 1H); 2.26(s, 3H); 2.12(s, 3H); 1.86(s, 3H); 1.64(t, 1H); 1.52(s, 3H); 1.06(s, 3H); 1.02(s, 3H).

### 27.2 Synthesis of [poly-(L)-glutamic acid]-[2'-O-(phenylalanyl)-paclitaxel] conjugate (I₂₇)

I₂₇ was prepared by following the procedure as described in Example 3.2, except for replacing 2'-O-(glycyl)-paclitaxel with 2'-O-(L-phenylalanyl)-paclitaxel in the condensation with the poly-(L)-glutamic acid under the action of dicyclohexylcarbodiimide. The content of paclitaxel in the conjugate I₂₇ was found as 26.6wt%.

### Example 28 Preparation of [N-acetyl-poly-(L)-glutamic acid]-[2'-O -(L-phenylalanyl)-paclitaxel] conjugate (I₂₈)

I₂₈ was prepared by following the procedure as described in Example 4, except for replacing 2'-O-(glycyl)-paclitaxel with 2'-O-(L-phenylalanyl)-paclitaxel in the reaction with the active ester of the N-acetyl-poly-(L)-glutamic acid. The content of paclitaxel in the conjugate was found as 24.5wt%.

### Example 29 Preparation of [poly-(L)-glutamic acid]-[2'-O-(L-phenylalanyl) -docetaxel] conjugate (I₂₉)

### 29.1 Synthesis of 2'-O-(L-phenylalanyl)-docetaxel

2'-O-(N-benzyloxycarbonyl-L-phenylalanyl)-docetaxel was prepared by following the procedure as described in Example 5.1, except for replacing N-benzyloxycarbonyl -glycine with N-benzyloxycarbonyl-(L)-phenylalanine in the condensation with docetaxel. Catalytic hydrogenation was performed to remove the protection group, to give 2'-O-(L-phenylalanyl)-docetaxel. ¹HNMR: δ (ppm, DMSO-d₆): 9.25(d, 1H); 8.48(brs, 2H); 8.05-7.32(m, 15H); 6.38(s, 1H); 5.80(t, 1H); 5.57(t, 1H); 5.42(d, 1H); 5.36(d, 1H); 4.94(dd, 1H); 4.82(brs, 1H); 4.61(s, 1H); 4.52(m, 1H); 4.14(dd, 1H); 4.01(d, 1H); 4.04(d, 1H); 3.57(d, 1H); 3.15(dd, 1H); 3.01(dd, 1H); 2.34(m, 1H); 2.24(s, 3H); 1.86(s, 3H); 1.80(dd, 1H); 1.64(t, 1H); 1.52(s, 3H); 1.40(s, 9H); 1.06(s, 3H); 1.02(s, 3H).

### 29.2 Synthesis of [poly-(L)-glutamic acid]-[2'-O-(phenylalanyl)-docetaxel] conjugate (I₂₉)

I₂₉ was prepared by following the procedure as described in Example 3.2, except for replacing 2'-O-(glycyl)-paclitaxel with 2'-O-(L-phenylalanyl)-docetaxel in the condensation with the poly-(L)-glutamic acid under the action of dicyclohexylcarbodiimide. The content of docetaxel in the conjugate I₂₉ was found as 24.0wt%.

### Example 30 Preparation of [N-acetyl-poly-(L)-glutamic acid]-[2'-O -(L-phenylalanyl)-docetaxel] conjugate (I₃₀)

I₃₀ was prepared by following the procedure as described in Example 4, except for replacing 2'-O-(glycyl)-paclitaxel with 2'-O-(L-phenylalanyl)-docetaxel in the reaction with the active ester of the N-acetyl-poly-(L)-glutamic acid. The content of paclitaxel in the conjugate was found as 23.8wt%.

### Example 31 Evaluation of activities against transplanted human liver cancer tumor in mice

Balb/c-nu/nu nude mice (5 to 7 weeks old, 16-20 g) were randomly grouped. The mice were inoculated subcutaneously with 0.1 ml of a cell suspension containing 10⁶ human liver cancer cells (HepG2). Upon the tumor volume having reached 50 mm³, the mice were intravenously injected with a dose of a conjugate or a free drug. Twenty days after the administration, the animals were sacrificed and their body weight and tumor weight were measured. Tumor inhibition rate was calculated according to the following equation:

Tumor inhibition rate (%) = [(average weight of tumor for the control group - average weight of tumor for the group administrated with the drug) / average weight of tumor for the control group] x 100%

The experimental results are shown in Table 1 below.

**Table 1 Inhibition effect of the target compounds to the transplanted human liver cancer tumor**

| Compound | Dose (mg/kg) | Number of animals (final/initial) | Change of body weight (g) | Weight of tumor (g) | Inhibition rate (%) |
|---|---|---|---|---|---|
| Physiological saline | | 6/6 | +9.32 | 1.26 | |
| Gemcitabine | 5 | 4/6 | +4.77 | 0.45 | 64.28 |
| I₁ | 5 | 6/6 | +9.94 | 0.31 | 75.40 |
| I₈ | 5 | 6/6 | +8.77 | 0.67 | 46.82 |
| Paclitaxel | 100 | 5/6 | +5.38 | 0.54 | 57.14 |
| I₄ | 100 | 6/6 | +9.03 | 0.36 | 71.43 |
| I₁₀ | 100 | 6/6 | +8.69 | 0.21 | 83.33 |
| I₂₂ | 100 | 6/6 | +7.35 | 0.47 | 62.70 |
| Docetaxel | 100 | 6/6 | +6.92 | 0.56 | 55.56 |
| I₆ | 100 | 6/6 | +8.49 | 0.32 | 74.60 |
| I₁₂ | 100 | 6/6 | +9.00 | 0.44 | 65.08 |
| I₁₈ | 100 | 6/6 | +8.75 | 0.38 | 69.84 |

The administrated doses have been converted into amounts of free drugs.

Example 32 Evaluation of activities against transplanted human breast cancer tumor in mice

Balb/c-nu/nu nude mice (5 to 7 weeks old, 16-20 g) were randomly grouped. The mice were inoculated subcutaneously with 0.1 ml of a cell suspension containing 10⁶ human breast cancer cells (Bacp-37). Upon the tumor volume having reached 50 mm³, the mice were intravenously injected with a dose of a conjugate or a free drug. Twenty days after the administration, the animals were sacrificed and their body weight and tumor weight were measured. The tumor inhibition rates were calculated and the results are shown in Table 2 below.

**Table 2 Inhibition effect of the target compounds to the transplanted human breast cancer tumor**

| Compound | Dose (mg/kg) | Number of animals (final/initial) | Change of body weight (g) | Weight of tumor (g) | Inhibition rate (%) |
|---|---|---|---|---|---|
| Physiological saline | | 6/6 | +9.76 | 1.38 | |
| Gemcitabine | 5 | 5/6 | +5.08 | 0.51 | 63.08 |
| I₁ | 5 | 6/6 | +9.42 | 0.46 | 71.01 |
| I₈ | 5 | 6/6 | +8.25 | 0.39 | 71.73 |
| Paclitaxel | 100 | 5/6 | +5.91 | 0.48 | 65.22 |
| I₄ | 100 | 6/6 | +9.24 | 0.42 | 69.56 |
| I₁₀ | 100 | 6/6 | +9.06 | 0.29 | 78.99 |
| I₂₂ | 100 | 6/6 | +8.30 | 0.41 | 70.29 |
| Docetaxel | 100 | 6/6 | +6.29 | 0.35 | 74.64 |
| I₆ | 100 | 6/6 | +10.12 | 0.47 | 65.94 |
| I₁₂ | 100 | 6/6 | +8.86 | 0.34 | 75.36 |
| I₁₈ | 100 | 6/6 | +9.16 | 0.73 | 47.10 |

It can be seen from the Table 1 and Table 2 that, compared with the free drugs, the target compounds exhibit comparable or better inhibition effect on solid tumors, whereas they affect the body weight less, indicating that the target compounds have lower toxicity and side effects.

## Claims

1. A conjugate formed by coupling gemcitabine, paclitaxel or docetaxel as an antitumor drug with a poly-(L)-glutamic acid via an amino acid, or a non-toxic pharmaceutically acceptable salt thereof.

2. The conjugate or the non-toxic pharmaceutically acceptable salt thereof according to claim 1, which is a conjugate of gemcitabine with a poly-(L)-glutamic acid coupled with an amino acid, or a non-toxic pharmaceutically acceptable salt thereof, wherein the α-position carboxyl group in the amino acid molecule forms an ester linkage with the 5'-position hydroxyl group in the gemcitabine molecule, the α-position amino group in the amino acid molecule forms an amide linkage with the carboxyl group in the poly-(L)-glutamic acid molecule, and the content of gemcitabine in the conjugate is in a range of from 5 to 30 wt%.

3. The conjugate or the non-toxic pharmaceutically acceptable salt thereof according to claim 1, which is a conjugate of paclitaxel or docetaxel with a poly-(L)-glutamic acid coupled with an amino acid, or a non-toxic pharmaceutically acceptable salt thereof, wherein the α-position carboxyl group in the amino acid molecule forms an ester linkage with the 2 '-position hydroxyl group in the paclitaxel or docetaxel molecule, the α-position amino group in the amino acid molecule forms an amide linkage with the carboxyl group in the poly-(L)-glutamic acid molecule, and the content of paclitaxel or docetaxel in the conjugate is in a range of from 10 to 40wt%.

4. The conjugate formed by coupling gemcitabine, paclitaxel or docetaxel with a poly-(L)-glutamic acid via an amino acid or the non-toxic pharmaceutically acceptable salt thereof according to claim 1, 2 or 3, wherein the poly-(L)-glutamic acid has a molecular weight of from 25, 000 to 50, 000.

5. The conjugate formed by coupling gemcitabine, paclitaxel or docetaxel with a poly-(L)-glutamic acid via an amino acid or the non-toxic pharmaceutically acceptable salt thereof according to claim 1, 2, 3 or 4, wherein the poly-(L)-glutamic acid molecule has a free amino or an alkanoylated amino at its N-terminal.

6. The conjugate formed by coupling gemcitabine, paclitaxel or docetaxel with a poly-(L)-glutamic acid via an amino acid or the non-toxic pharmaceutically acceptable salt thereof according to claim 1, 2, 3, 4 or 5, wherein the amino acid is glycine, (L)-alanine, (L)-leucine, (L)-isoleucine, (L)-valine, (L)-proline, (L)-phenylalanine, (L)-methionine or (L)-glutamine.

7. A pharmaceutical composition comprising the conjugate formed by coupling gemcitabine, paclitaxel or docetaxel with a poly-(L)-glutamic acid via an amino acid or the non-toxic pharmaceutically acceptable salt thereof according to claim 1, 2, 3, 4, 5 or 6 as an active component.

8. Use of the conjugate formed by coupling gemcitabine, paclitaxel or docetaxel with a poly-(L)-glutamic acid via an amino acid or the non-toxic pharmaceutically acceptable salt thereof according to claim 1, 2, 3, 4, 5 or 6 in the preparation of an antitumor drug.
